(19) Europäisches Patentamt / European Patent Office / Office européen des brevets

(11) **EP 3 831 357 B1**

(12) **FASCICULE DE BREVET EUROPEEN**

(45) Date de publication et mention de la délivrance du brevet:
**17.04.2024 Bulletin 2024/16**

(21) Numéro de dépôt: **20212028.3**

(22) Date de dépôt: **04.12.2020**

(51) Classification Internationale des Brevets (IPC):
**A61K 8/02** *(2006.01)*  **A61K 8/06** *(2006.01)*
**A61K 8/36** *(2006.01)*  **A61K 8/73** *(2006.01)*
**A61K 8/81** *(2006.01)*  **A61Q 1/10** *(2006.01)*

(52) Classification Coopérative des Brevets (CPC):
**A61K 8/0241; A61K 8/062; A61K 8/361;**
**A61K 8/73; A61K 8/8147; A61Q 1/10;**
A61K 2800/436; A61K 2800/612

(54) **COMPOSITION COSMÉTIQUE À EFFET MÉTALLIQUE**

KOSMETISCHE ZUBEREITUNG MIT METALLEFFEKT

COSMETIC COMPOSITION WITH METALLIC EFFECT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.12.2019 FR 1913897**

(43) Date de publication de la demande:
**09.06.2021 Bulletin 2021/23**

(73) Titulaire: **Chanel Parfums Beauté**
**92200 Neuilly-sur-Seine (FR)**

(72) Inventeur: **BONNEFOY, Aurélie**
**93694 PANTIN CEDEX (FR)**

(74) Mandataire: **Plasseraud IP**
**66, rue de la Chaussée d'Antin**
**75440 Paris Cedex 09 (FR)**

(56) Documents cités:
**EP-A1- 3 162 359**    **FR-A1- 3 006 188**
**US-A1- 2004 223 929**    **US-A1- 2017 007 512**

Remarques:
Le dossier contient des informations techniques présentées postérieurement au dépôt de la demande et ne figurant pas dans le présent fascicule.

**Description**

La présente invention a pour objet une composition cosmétique comprenant, dans une phase continue aqueuse, au moins un agent de coloration choisi parmi les nacres traitées hydrophobes, au moins un gélifiant hydrophile, et de l'eau. L'invention se rapporte également à un procédé de préparation d'une telle composition cosmétique, à un procédé de maquillage des matières kératiniques la mettant en oeuvre et à l'utilisation de cette composition cosmétique pour l'obtention d'un effet métallique aqueux ou « effet miroir » sur les matières kératiniques.

Les produits de maquillage ou de traitement des matières kératiniques tels que les fonds de teint, les fards à joues ou les fards à paupières, ont généralement pour fonction d'apporter de la couleur, de la matité ou encore conférer de la couvrance.

D'une manière générale, ces produits de maquillage, sont majoritairement constitués d'une phase continue huileuse dans laquelle la quantité des charges et d'agents de coloration est modulée afin de procurer les effets de maquillage recherchés : coloriel, couvrant, matifiant...

Les effets métallisés, irisés ou pailletés sont par ailleurs très recherchés pour les produits de maquillage. Pour obtenir de tels effets coloriels, il est connu de mettre en oeuvre des pigments à effet optique tels que des nacres ou autres pigments interférentiels.

Pour améliorer leur dispersion dans les produits à phase continue huileuse, il est connu de traiter en surface les agents coloriels au moyen d'un revêtement hydrophobe, ce qui garantit une couleur et un effet optique des plus homogènes possible, et permet notamment d'éviter les zones de surbrillance.

Si les effets coloriels ainsi obtenus sont intenses avec des couleurs saturées, et les effets métalliques satinés, ces formulations en phase continue huileuse procurent souvent une sensation de lourdeur ainsi qu'un effet collant à l'application, désagréable pour l'utilisateur.

Les formulations présentant une phase continue aqueuse sont, quant à elles, très prisées pour leur aspect sensoriel frais, léger, et non gras. Elles sont le plus souvent utilisées pour les produits de soins. En effet, les produits colorés obtenus à partir de formulation présentant une phase continue aqueuse sont généralement insuffisamment couvrants pour des produits de maquillage. Les couleurs manquent d'intensité et ne présentent pas une bonne tenue dans le temps. Il n'est en outre pas possible d'obtenir des effets coloriels particuliers tels qu'un effet métallique aqueux ou « effet miroir », à partir de telles galéniques.

Les agents de coloration particulaires tels que les pigments ou les nacres, ne se dispersent pas de manière homogène dans la phase aqueuse, et forment des agrégats susceptibles de poser des problèmes de stabilité. Il en résulte que très peu de produits de maquillage actuellement sur le marché présentent une phase aqueuse continue.

On demeure toutefois à la recherche de compositions cosmétiques de maquillage fraiches et confortables, présentant un effet coloriel intense avec des couleurs saturées tel qu'un effet métallique aqueux ou « effet miroir » ainsi qu'une bonne tenue dans le temps dudit effet métallique.

La demanderesse a découvert de façon inattendue qu'une composition présentant de telles propriétés, a priori non conciliables, pouvait être obtenue en utilisant des nacres traitées hydrophobes dans une phase continue hydrophile.

Les compositions selon l'invention permettent d'obtenir un effet coloriel de type métallique aqueux ou effet « miroir », tout en proposant une nouvelle texture longue tenue, fraiche et confortable.

L'invention a ainsi pour objet, selon un premier aspect, une composition cosmétique comprenant, dans une phase continue aqueuse :

- au moins un agent de coloration choisi parmi les nacres traitées hydrophobes ;
- au moins un gélifiant hydrophile ;
- de l'eau.

L'invention a également pour objet, selon un deuxième aspect, un procédé de préparation d'une telle composition, comprenant :

- le mélange du gélifiant hydrophile avec l'eau, et optionnellement avec les agents émulsionnants hydrocarbonés et/ou siliconés, les humectants et les solvants solubles dans l'eau ;
- optionnellement l'ajout des agents filmogènes, des agents émulsionnants siliconés et/ou hydrocarbonés et des additifs ;
- l'ajout des nacres traitées hydrophobes et optionnellement des pigments traités hydrophobes ;
- optionnellement l'ajustement du pH.

L'invention a encore pour objet, selon un troisième aspect, un procédé de maquillage des matières kératiniques, en particulier la peau ou les lèvres, consistant à appliquer sur lesdites matières kératiniques, en particulier la peau ou les lèvres, une composition telle que décrite précédemment.

Enfin, l'invention a pour objet l'utilisation cosmétique d'une composition telle que décrite précédemment pour former, sur les matières kératiniques, en particulier sur la peau ou les lèvres, un maquillage coloré à effet métallique aqueux ou « effet miroir ».

Ainsi, l'invention a pour objet une composition cosmétique comprenant, dans une phase continue aqueuse,

- au moins un agent de coloration choisi parmi les nacres traitées hydrophobes ;
- au moins un gélifiant hydrophile ;
- de l'eau.

Galénique

La composition selon l'invention présente une phase continue aqueuse. De préférence, elle se présente sous la forme d'un gel aqueux ou d'une émulsion huile-dans-eau.

Nacres traitées hydrophobes

Les nacres traitées hydrophobes sont présentes dans la phase continue aqueuse de la composition selon l'invention en une teneur allant de 16 à 35% en poids, de préférence de 20 à 33% en poids, et de façon encore plus préférée, la teneur en nacres traitées hydrophobes est d'environ 25% en poids par rapport au poids total de la composition.

La présence de ces nacres présentent dans la phase continue aqueuse permet avantageusement d'obtenir nouvel effet coloriel de type effet métallique aqueux.

Cet effet métallique est obtenu pour des teneurs en nacres traitées hydrophobes allant de 16 à 35%. En deçà de ces teneurs, l'effet coloriel métallique recherché n'est pas suffisamment visible. Au-delà, celui-ci est trop prononcé et devient trop brillant.

Les nacres peuvent être choisies parmi celles classiquement présentes dans les produits de maquillage, telles que les nacres à base de mica / dioxyde de titane (par exemple, DK PEARL SILVER 310 commercialisées par la société DAITO KASEI EUROPE), à base de mica / oxyde de titane / oxyde de fer (telles que les DK PEARL ORANGE GOLD, DK PEARL DARK RED ou DK PEARL GOLD commercialisées par la société DAITO KASEI EUROPE), à base de mica/oxydes de fer (par exemple DK PEAR BRONZE commercialisées par la société DAITO KASEI EUROPE) à base de mica / silice / dioxyde de titane, à base de fluorphlogopite synthétique / dioxyde de titane (SUNSHINE® de MAPRECOS), de calcium sodium borosilicate / dioxyde de titane (REFLECKS® d'ENGELHARD) ou de calcium aluminium borosilicate / silice / dioxyde de titane (RONASTAR® de MERCK).

Selon un mode de réalisation préféré, les nacres seront choisies parmi les nacres à base de mica / dioxyde de titane, à base de mica / oxyde de titane / oxyde de fer, à base de mica / oxydes de fer, et leurs mélanges.

Selon un mode de réalisation, les nacres sont des nacres traitées hydrophobes.

Selon un mode de réalisation, le traitement hydrophobe est un traitement par savon métallique.

Les nacres sont en effet au moins en partie, préférentiellement en totalité, traitées en surface par un savon métallique.

Typiquement, le savon métallique est un savon d'acides gras ayant de 12 à 22 atomes de carbone, et en particulier de 12 à 18 atomes de carbone.

Le métal du savon métallique est quant à lui de préférence choisi parmi le zinc et le magnésium.

Ainsi, selon un mode préféré de réalisation, le savon métallique est choisi parmi le laurate de zinc, le stéarate de magnésium, le myristate de magnésium, le stéarate de zinc, et leurs mélanges.

De préférence, le savon métallique est du stéarate de magnésium.

Gélifiant hydrophile

La phase continue aqueuse de la composition selon l'invention comprend au moins un gélifiant hydrophile.

Par gélifiant, on entend un composé qui, en présence d'un solvant, crée des liaisons inter-macromoléculaires plus ou moins fortes induisant ainsi un réseau tridimensionnel qui fige ledit solvant.

Le gélifiant est un gélifiant de phase aqueuse qui permet avantageusement de disperser les nacres traitées hydrophobes dans la phase aqueuse ainsi que de les maintenir en suspension.

Le gélifiant peut être choisi parmi les polysaccharides, les polyacrylates, les polyméthacrylates et leurs dérivés.

Parmi les polysaccharides, on peut citer :

- les exsudats de micro-organismes tels que la gomme de xanthane et ses dérivés comme le produit vendu sous la dénomination commerciale Rhéosan par la Société Rhodia Chimie, le produit vendu sous la dénomination KELTROL® CG-SFT (INCI : xanthan gum) par la société CP Kelco, ou le produit SAFIC CARE T XCG (INCI : Ceratonia siliqia gum & xanthan gum) par la société SAFIC ALCAN, la gomme de gellane vendue sous la dénomination commerciale Kelcogel F par la société NUTRASWEET-KELCO ou encore le iota carraghenane vendu sous les dénominations commerciales Seaspen PF 357 ou Viscarin SD 389 par la société FMC, ou la gomme de sclérotium (sclerotium gum ou gomme de sclerotium rolfssii), produite par la bactérie Sclerotium rolfissii, disponible sous la dénomination Naturajel® par la société DIY Cosmétics ou l'Amigel® par la société Alban Muller ;

- les extraits de végétaux tels que les polysaccharides de tremella fuciformis (INCI : Tremella fuciformis polysaccharide), comme le produit vendu sous la dénomination Tremoist™ -TP par la société Nippon Fine Chemical ;
- les extraits d'algue tels que l'agar-agar, les carraghénanes (iota, kappa, lambda) comme le produit vendu sous la dénomination Viscarin PC 209 (INCI : Carrageenan) par la société FMC BioPolymer, et leurs dérivés tels que le produit vendu sous la dénomination Sucraclear HC-31 (INCI : Cellulose Gum, Carrageenan, Ceratonia Siliqua Gum, Sucrose) par la société ALCHEMY ingrédients, les alginates, en particulier de Na ou Ca ;
- et leurs mélanges.

Parmi les polyacrylates, on peut citer :

- les polymères d'acide acrylique tel que le carbomère, comme celui vendu sous la dénomination Carbobol Ultrez 10 (INCI : carbomer), par les sociétés Lubrizol ou Gattefosse France,
- les polymères d'acrylate de méthyle et de méthacrylate de béhényle polyoxyéthyléné 25 OE réticulés (nom INCI : Acrylates/Beheneth-25 Méthacrylate Copolymer), tel que celui vendu sous la dénomination Novethix L-10 Polymer par la société Lubrizol Advanced Materials, Rheostyl™ 90 N d'Arkema, ou Aculyn™ 28 de la société Dow Chemical.

De préférence, le gélifiant est choisi parmi les polysaccharides tels que les exsudats de micro-organismes ou les polysaccharides extraits de végétaux, les polymères d'acide acrylique ou un de leurs mélanges.

De préférence encore, le gélifiant est choisi parmi le carbomère, la gomme xanthane, le tremella fuciformis polysaccharide, ou un de leurs mélanges.

Selon un mode de réalisation, la phase continue aqueuse de la composition selon l'invention comprend un seul gélifiant de phase aqueuse, préférentiellement le carbomère.

Selon un mode de réalisation, le gélifiant est présent en une teneur allant de 0.05% à 3% en poids, de préférence de 0.1 à 2% en poids, et de manière encore plus préférée, de 0.3 à 2% en poids, par rapport au poids total de la composition.

Phase aqueuse

La composition selon l'invention comprend une phase aqueuse comprenant de l'eau.

Selon un mode particulier de réalisation, la composition selon l'invention comprend de 30 à 70% en poids d'eau, de préférence de 40 à 60% en poids, et de manière encore plus préférée, environ 50% en poids d'eau, par rapport au poids total de la composition.

Les teneurs en eau de la composition selon l'invention permettent avantageusement de conférer à la composition cosmétique un effet frais immédiat. Une fois appliquée, la composition cosmétique selon l'invention est légère, sans sensation de lourdeur ni collante. Il est ainsi possible d'obtenir une composition cosmétique de maquillage fraiche et confortable.

Solvants solubles dans l'eau

La composition selon l'invention comprend en outre, dans la phase continue aqueuse, au moins un solvant soluble dans l'eau.

Par « solvant soluble dans l'eau », on désigne un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent être volatils.

Parmi les solvants hydrosolubles pouvant être utilisés dans les compositions conformes à l'invention, on peut citer les mono-alcools ayant de 1 à 5 atomes de carbone tels que l'éthanol et l'isopropanol, les cétones en $C_3$-$C_4$ et les aldéhydes en $C_2$-$C_4$.

Ainsi, selon un mode préféré de réalisation, la composition selon l'invention comprenant au moins un mono-alcool ayant de 1 à 5 atomes de carbone, de préférence l'éthanol.

Typiquement, le mono-alcool ayant de 1 à 5 atomes est présent en une teneur allant de 0,1 à 10% en poids, par rapport au poids total de la composition.

L'introduction d'un mono-alcool ayant de 1 à 5 atomes de carbone permet de faciliter et d'accélérer le séchage de la composition.

Humectants

La composition selon l'invention comprend au moins un humectant.

Parmi les humectants, on citera notamment les polyols.

Ainsi, la composition selon l'invention comprend en outre un humectant choisi parmi les polyols.

On entend par polyol toute molécule organique présentant dans sa structure au moins 2 groupements hydroxy (-OH) libres. Ces polyols sont de préférence liquides à température ambiante (25°C).

A titre d'exemple, les polyols convenant à la mise en oeuvre dans la composition peuvent être choisis parmi le propylène glycol, le butylène glycol, le pentylène glycol, le pentanediol, l'isoprène glycol, le néopentyl glycol, le glycérol, les polyéthylène glycols (PEG) ayant notamment de 4 à 8 motifs éthylène glycol et/ou le sorbitol.

De préférence, l'humectant est choisi parmi le glycérol et le butylène glycol.

Selon un mode particulier de réalisation, la composition selon l'invention comprend de 0 à 20% en poids d'humectant, de préférence, de 5 à 10% en poids d'humectant par rapport au poids total de la composition, et de manière encore plus préférée, la composition selon l'invention comprend environ 6% en poids d'humectant par rapport au poids total de la composition.

Pigments

La composition selon l'invention comprend en outre, dans sa phase aqueuse, au moins un autre agent de coloration choisi parmi les pigments traités hydrophobes, dans la mesure où la teneur en pigment traités hydrophobes par rapport au poids total de la composition reste inférieure à 1,5%.

Avantageusement, la demanderesse a démontré qu'il est possible d'introduire des pigments traités hydrophobes, en une teneur inférieure à 1,5% en poids, par rapport au poids total de la composition, dans les compositions en phase continue aqueuse selon l'invention afin de renforcer l'éclat et l'effet coloriel, et d'éviter que les couleurs ne ternissent, et ce, sans déstabiliser la composition. Plus particulièrement, la demanderesse a démontré qu'il est possible d'obtenir des gels de carbomère contenant des oxydes de fer pour apporter de la couleur sans déstabiliser le gel.

Selon un mode de réalisation, la composition est exempte de pigments traités hydrophobes.

Par « pigments », il faut comprendre des particules blanches ou colorées, minérales ou organiques, insolubles dans un milieu aqueux, destinées à colorer et/ou opacifier la composition.

Les pigments peuvent être blancs ou colorés, minéraux et/ou organiques.

Le pigment peut être un pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références C1 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI11725, 15510,45370, 71105,les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque. Par laque, on entend les colorants insolubilisés adsorbés sur des particules insolubles, l'ensemble ainsi obtenu restant insoluble lors de l'utilisation. A titre d'exemples de laques, on peut citer le produit connu sous la dénomination suivante : D & C Red 7 (CI 15 850:1).

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : $Ti_2O_5$, $Ti_3O_5$, $Ti_2O_3$, $TiO$, $ZrO_2$ en mélange avec $TiCO_2$, $ZrO_2$, $Nb_2O_5$, $CeO_2$, $ZnS$.

Selon un mode de réalisation préféré, le pigment est un pigment minéral tel qu'un oxyde de fer, préférentiellement un oxyde de fer noir.

La taille du pigment utile dans le cadre de la présente invention est généralement comprise entre 10 nm et 10 µm, de préférence entre 20 nm et 5 µm, et plus préférentiellement entre 30 nm et 1 µm.

Selon un mode de réalisation, le traitement hydrophobe des pigments est un traitement par savon métallique.

Les pigments sont en effet au moins en partie, préférentiellement en totalité, traités en surface par un savon métallique.

Typiquement, le savon métallique est un savon d'acides gras ayant de 12 à 22 atomes de carbone, et en particulier de 12 à 18 atomes de carbone.

Le métal du savon métallique est quant à lui de préférence choisi parmi le zinc et le magnésium.

Ainsi, selon un mode préféré de réalisation, le savon métallique est choisi parmi le laurate de zinc, le stéarate de magnésium, le myristate de magnésium, le stéarate de zinc, et leurs mélanges.

De préférence, le savon métallique est du stéarate de magnésium.

Charges

La composition selon l'invention peut également comprendre au moins une charge. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, le mica traité en surface par un agent hydrophobe, la cellulose, la cellulose traitée en surface par un agent hydrophobe, la silice, la silice traitée en surface par un agent hydrophobe, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société DOWSIL ) et les microbilles de résine de silicone (Tospearls® de Toshiba, par exemple), les particules de polyorganosiloxanes élastomères, le carbonate de calcium précipité, le carbonate et l'hydra-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc de magnésium ou de lithium, le laurate de zinc, le myristate de magnésium.

Selon un mode de réalisation, la charge est un mica traité hydrophobe par un traitement par savon métallique, préférentiellement un savon_d'acides gras ayant de 12 à 22 atomes de carbone, et en particulier de 12 à 18 atomes de carbone. Préférentiellement, le métal du savon métallique est du zinc ou du magnésium.

A titre illustratif, on citera le MICA SX commercialisé par la société DAITO KASEI EUROPE.

Agent filmogène

La composition selon l'invention comprend en outre un agent filmogène, en particulier un polymère filmogène.

Selon un premier mode de réalisation, le polymère filmogène peut être présent dans une composition de l'invention sous la forme de particules en dispersion dans une phase aqueuse. On parle de polymère filmogène dispersible en phase aqueuse, connu généralement sous le nom de latex ou pseudolatex. Les techniques de préparation de ces dispersions sont bien connues de l'homme du métier.

Comme polymère filmogène dispersible en phase aqueuse ou dispersion aqueuse de polymère filmogène, on peut utiliser les dispersions acryliques vendues sous les dénominations Neocryl XK-90®, Neocryl A-10700, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO ; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-4100, Avalure UR-425®, Avalure UR-450®, Sancure 875®, Sancure 8610, Sancure 878® et Sancure 2060® par la société GOODRICH, Impranil 85® par la société BAYER, Aquamere H-15110 par la société HYDROMER ; les sulfopolyesters vendus sous le nom de marque Eastman AQ® par la société Eastman Chemical Products, les dispersions vinyliques comme le Mexomère PAM® de la société CHLMEX et leurs mélanges.

Selon un deuxième mode de réalisation, le polymère filmogène peut être un polymère hydrosoluble et peut être alors présent dans la phase continue aqueuse de la composition selon l'invention.

Selon un troisième mode de réalisation, le polymère filmogène peut être un polymère solubilisé dans une phase grasse liquide comprenant des huiles ou solvants organiques. on dit alors que le polymère filmogène est un polymère filmogène lipophile (liposoluble ou lipodispersible).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyle éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux allyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de méthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200000.

On peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly(méth)acrylates de lauryle, ces poly(méth)acrylates pouvant être réticulés à l'aide de diméthacrylate de l'éthylène glycol ou de tétraéthylène glycol.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1à C8comme l'éthylcellulose et la propylcellulose, les copolymères de la vinylpyrrolidone (VP) et notamment les copolymères de la vinylpyrrolidone et d'alcène en C2à C40et mieux en C3à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécene, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomériques siloxane qu'elle comprend, chacune des lettres « MDTQ » caractérisant un type d'unité.

A titre d'exemples de résines polyméthylsilsesquioxanes commercialement disponibles, on peut citer celles qui sont commercialisés par la société Wacker Chemie AG sous la référence Resin MK tels que la Belsil PMS MK, et par la société SHIN-ETSU sous les références KR-220L

Comme résines siloxysilicates, on peut citer les résines triméthylsiloxysilicate (TMS) telles que celles commercialisées sous la référence SR1000 par la société General Electric ou sous la référence Belsil TMS 803 par la société Wacker Chemie AG. On peut encore citer les résines triméthylsiloxysilicate commercialisées dans un solvant tel que la cyclomethicone, vendues sous la dénomination KF-7312J par la société Shin-Etsu, DOWSIL™ RSN-0749, DOWSIL™ 593 Fluid par la société DOWSIL. On citera également les dispersions aqueuses de résines triméthylsiloxysilicate, vendues sous la dénomination GRANRESIN SIW-MQIZ (INCI : Isododecane (and) Trimethylsiloxysilicate (and) Aqua (and) Propanediol (and) Decyl Glucoside) commercialisées par la société GRANT INDUSTRIES.

On peut aussi citer des copolymères de résines de silicone telles que celles citées ci-dessus avec des polydiméthylsiloxanes, comme les copolymères adhésifs sensibles à la pression commercialisés par la société DOWSIL sous la référence BIO-PSA et décrits dans le document US 5,162,410 ou encore les copolymères siliconés issus de la réaction d'un résine de silicone, telle que celles décrite plus haut, et d'un diorganosiloxane tels que décrits dans le document WO 2004/073626.

On peut également citer les copolymères acrylate/polytriméthylsiloxyméthacrylate comprenant une structure carbosiloxane dendrimère greffée sur un squelette vinylique disponible dans le commerce sous les références DOWSIL FA 4002 ID ou DOWSIL FA 4001 CM, ou DOWSIL FA 4103 (dispersion aqueuse).

On peut enfin utiliser les polyamides siliconés du type polyorganosiloxane tels que ceux décrits dans les documents US-A-5.,874,069, US-A-5,919,441, US-A-6,051,216 et US-A-5,981,680.

Selon un mode préféré de réalisation, le polymère filmogène est choisi parmi les polymères d'origine naturelle, éventuellement modifiées, de préférence parmi les polymères extraits du fruit de *Caesalpinia spinosa* et/ou de l'algue *Kappaphycus alvarezii* (tel que le produit Filmexel® commercialisé par la société Silab).

Selon un mode de réalisation préféré, l'agent filmogène est choisi parmi un polymère filmogène dispersible en phase aqueuse, préférentiellement, une dispersion aqueuse de résines triméthylsiloxysilicate, vendue sous la dénomination GRANRESIN SIW-MQIZ (INCI : Isododecane (and) Trimethylsiloxysilicate (and) Aqua (and) Propanediol (and) Decyl Glucoside)) commercialisées par la société GRANT INDUSTRIES, un polymère filmogène liposoluble tel que les résines triméthylsiloxysilicate telles que celles commercialisées sous la référence Belsil TMS 803 par la société Wacker Chemie AG dispersées dans l'isododecane, les copolymères acrylate/polytriméthylsiloxyméthacrylate comprenant une structure carbosiloxane dendrimère greffée sur un squelette vinylique disponible dans le commerce sous les références DOWSIL FA 4002 ID, et leurs mélanges.

Selon un mode de réalisation, l'agent filmogène est présent dans la composition selon l'invention en un teneur comprise entre 5 et 20% en poids, préférentiellement, entre 5 et 15% en poids, et de manière encore plus préférée, entre 10 et 15% en poids, par rapport au poids total de la composition.

Ainsi, les compositions cosmétiques selon l'invention, outre l'effet métallique ainsi que la fraicheur et le confort à l'application, offrent également une longue tenue après application grâce au filmogène utilisé.

On entend par longue tenue, une composition cosmétique qui ne s'estompe pas ou très légèrement, qui ne perd pas ou très légèrement en homogénéité, qui ne migrent pas et ne diffuse pas après application et ce pendant une durée de au moins 3h30, préférentiellement au moins 7h30.

Phase Huileuse

La composition selon l'invention peut comprendre au moins une huile et au moins un agent émulsionnant, ladite composition étant une émulsion huile-dans-eau.

Selon un mode de réalisation, l'huile sera choisie parmi les huiles volatiles et/ou les huiles non volatiles, et leurs mélanges.

Selon un mode de réalisation préféré, l'huile est une huile volatile.

Par « huile volatile », on entend au sens de l'invention une huile susceptible de s'évaporer au contact des fibres kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Selon un mode de réalisation, l'huile volatile est choisie parmi les huiles volatiles hydrocarbonées, les alcanes linéaires volatiles, et les huiles siliconées volatiles.

L'huile volatile peut être hydrocarbonée. L'huile volatile hydrocarbonée peut être choisie parmi les huiles hydrocarbonées ayant de 7 à 16 atomes de carbone. Comme huile volatile hydrocarbonée ayant de 7 à 16 atomes de carbone, on peut citer notamment les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée ayant de 8 à 16 atomes de carbone est choisie parmi l'isododécane, l'isodécane, l'isohexadécane et leurs mélanges, et est notamment l'isododécane.

L'huile volatile peut être un alcane linéaire volatil. Selon un mode de réalisation, un alcane convenant à l'invention peut être un alcane linéaire volatil comprenant de 7 à 14 atomes de carbone. Un tel alcane linéaire volatil peut être avantageusement d'origine végétale. A titre d'exemple d'alcanes convenant à l'invention, on peut mentionner les alcanes décrits dans les demandes de brevets de la société Cognis WO 2007/1068371, ou WO2008/155059 (mélanges d'alcanes distincts et différant d'au moins un carbone). Ces alcanes sont obtenus partir d'alcools gras, eux-mêmes obtenus à partir d'huile de coprah ou de palme. A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges. Selon un mode préféré, on peut citer les mélanges de n-undécane (C11) et de n-tridécane (C13) obtenus aux exemples 1 et 2 de la demande WO2008/15505 de la Société Cognis. On pourra également citer le mélange de n-undécane (C11) et de n-tridécane (C13) commercialisé par la société BASF sous le nom de CETIOL ULTIMATE. On peut encose citer le n-dodécane (C12) et le n-tétradécane (C14) vendus par Sasol respectivement sous les références PARAFOL 12-97 et PARAFOL 14-97, ainsi que leurs mélanges. On pourra utiliser l'alcane linéaire volatil seul ou préférentiellement un mélange d'au moins deux alcanes linéaires volatils distincts, différant entre eux d'un nombre de carbone n d'au moins 1, en particulier différant entre eux d'un nombre de carbone de 1 ou de 2.

L'huile volatile peut être une huile siliconée volatile telle que les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane, le dodecamethylcyclohexasiloxane, le cyclopentasiloxane.

Selon un mode de réalisation, l'huile volatile est présente en une teneur allant de 0 à 20% en poids, de préférence 3 à 10% en poids, par rapport au poids total de la composition.

Par « huile non volatile », on entend une huile restant sur les fibres kératiniques à température ambiante et pression atmosphérique au moins plusieurs heures et ayant notamment une pression de vapeur inférieure à $10^{-3}$mm de Hg (0,13 Pa).

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les alcanes linéaires en C4 à C36, de préférence C11-C21 comme le phyto squalane ou l'Emogreen L15 de SEPPIC (alcane en C15-19), ou encore telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de C4 à C36, et, notamment, de C18 à C36 ; ces huiles pouvant être linéaires ou ramifiées, saturées ou insaturées ; ces huiles peuvent, notamment, être des triglycérides héptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol, l'huile de ricin, et l'huile de watermelon, et leurs mélanges, ou encore des triglycérides d'acides caprylique/caprique, comme ceux vendus par la société STEARINERIES DUB0IS ou ceux vendus sous les dénominations MIGLYOL 8100, 8120 et 8180 par la société DYNAMIT NOBEL,
- les éthers de synthèse ayant de 10 à 40 atomes de carbone ;
- les esters de synthèse, comme les huiles de formule R1 COOR2, dans laquelle R1 représente un reste d'un acide gras linéaire ou ramifié comportant de 1 à 40 atomes de carbone et R2 représente une chaîne hydrocarbonée, notamment, ramifiée contenant de 1 à 40 atomes de carbone à condition que R1 + R2 soit $\geq$ 10. Les esters peuvent être, notamment, choisis parmi les esters d'alcool et d'acide gras, comme par exemple l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylène glycol et leurs mélanges, les benzoates d'alcools en C12 -CI5, le laurate d'hexyle, les esters de l'acide néopentanoïque, comme le néopentanoate d'isodécyle, le néopentanoate d'isotridécyle, le néopentanoate d'isostéaryle, le néopentanoate d'octyldocécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;

- les esters de polyols et les esters du pentaérythritol, comme le tétrahydroxystéarate/tétraisostéarate de dipentaérythritol,
- les esters de dimères diols et de dimères diacides, tels que les Lusplan DD-DA5® et Lusplan DD-DA7®, commercialisés par la société NIPPON FINE CHEMICAL et décrits dans la demande US 2004-175338,
- les copolymères de dimère diol et de dimère diacide et leurs esters, tels que les copolymères dimères dilinoleyl diol/dimères dilinoléiques et leurs esters, comme par exemple le Plandool-G,
- les copolymères de polyols et de dimères diacides, et leurs esters, tels que le Hailuscent ISDA,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique, le 2-hexyldécanol, le 2-blatyloctanol, et le 2-undécylpentadécanol,
- les acides gras supérieurs en $C_{12}$-$C_{22}$, tels que l'acide oléique, l'acide linoléique, et leurs mélanges,
- les carbonates de di-alkyle, les 2 chaînes alkyles pouvant être identiques ou différentes, tels que le dicaprylyl carbonate commercialisé sous la dénomination CETIOL CC®, par COGNIS,
- les huiles de masse molaire élevée ayant, en particulier, une masse molaire allant d'environ 400 à environ 10 000 g/mol, en particulier, d'environ 650 à environ 10 000 g/mol, en particulier, d'environ 750 à environ 7500 g/mol, et plus particulièrement, variant d'environ 1000 à environ 5000 g/mol,
- les huiles siliconées, telles que les silicones phénylées comme la BELSIL PDM 1000 de la société WACIER (MM=9000 g/mol). D'autres huiles de silicone non volatiles utilisables dans la composition selon l'invention peuvent être les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phénol triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges,
- les huiles fluorées utilisables dans l'invention sont notamment des huiles fluorosiliconées, des polyéthers fluorés, des silicones fluorées telles que décrit dans le document EP-A-847752.

Selon un mode de réalisation, la composition cosmétique selon l'invention comprend moins de 5% en poids d'huiles non volatiles par rapport au poids total de la composition, et de préférence, la composition est exempte d'huiles non volatiles.

Avantageusement, une teneur en huiles non volatiles inférieure à 5% en poids par rapport au poids total de la composition et préférentiellement une composition exempte d'huiles non volatiles permet d'obtenir une composition ayant une tenue améliorée.

Agent émulsionnant

L'agent émulsionnant peut être choisi parmi les émulsionnants hydrocarbonés, les émulsionnants siliconés, ou leurs mélanges.

Parmi les émulsionnants hydrocarbonés, on peut citer notamment :

- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 notamment vendu sous la dénomination Tween 20® par la société CRODA, le polysorbate 60 notamment vendu sous la dénomination Tween 60@ par la société CRODA.

Parmi les émulsionnants siliconés, on peut utiliser ceux du nom INCI PEG-10 Dimethicone, comme ceux commercialisés par la société DKSH France ou par la société Shin-Etsu Silicones, sous le nom KF-6017.

Selon un mode de réalisation préféré, l'agent émulsionnant est choisi parmi le PEG-10 Dimethicone, le polysorbate 20 ou polysorbate 60, ou leurs mélanges.

Selon un mode de réalisation, l'agent émulsionnant est présent en une teneur allant de 0.05 à 3% en poids, de préférence 0.1 à 2% en poids, par rapport au poids total de la composition et plus préférentiellement de 0.3 à 1 % en poids par rapport au poids total de la composition.

Effet coloriel métallique aqueux

La composition selon l'invention permet d'obtenir un effet coloriel métallique aqueux ou « effet miroir » dans des compositions présentant une phase continue aqueuse.

On entend par effet coloriel métallique aqueux, un effet optique offrant à la couleur un effet brillant, homogène, reflétant la lumière, tel un effet « miroir » sans toutefois être pailleté, ni satiné, ni présenter des « points de surbrillance ».

Cet effet optique est mesurable et quantifiable selon toute méthode connue de l'homme du métier.

Typiquement, les effets optiques sont mesurables et quantifiables par la méthode connue de l'homme du métier, la méthode de Réflexion au samba.

Cette méthode permet de mesurer l'intégrale spéculaire et le Wvisual, qui permettent de quantifier et évaluer l'apparence visuelle en termes de brillance, de couleur et de diffusion de la lumière.

Selon un mode de réalisation, la composition cosmétique selon l'invention présente une intégrale spéculaire supérieure ou égale à 6000 et un Wvisual supérieur ou égal à 8.

Préférentiellement, la composition cosmétique selon l'invention présente une intégrale spéculaire supérieure ou égale à 7000 et un Wvisual supérieur ou égal à 9.

Une intégrale spéculaire supérieure ou égale à 6000 et un Wvisual supérieur ou égal à 8, caractérisent l'effet métallique recherché dans le cadre de la présente invention, c'est-à-dire un effet métallique aqueux ou « effet miroir ».

L'effet métallique obtenu selon la présente invention est à distinguer d'un effet brillant, d'un effet satiné, ou d'un effet « point de surbrillance ».

Typiquement, une intégrale spéculaire approximativement égale à 5000, typiquement entre 4500 et 5500 et un effet Wvisual compris entre 4 et 7, approximativement 6 seront caractéristiques d'un effet brillant.

De la même façon, une intégrale spéculaire inférieure à 2500 avec un Wvisual inférieur ou égal à 3 seront caractéristiques d'un effet satiné.

Additifs

La composition selon l'invention peut comprendre d'autres ingrédients pour autant qu'ils n'interfèrent pas avec les propriétés souhaitées de la composition. Ces autres ingrédients peuvent par exemple être des conservateurs, des ajusteurs de pH tels l'acide citrique, la soude ou l'arginine, des agents antimicrobiens, des parfums, des filtres solaires, et leurs mélanges.

Procédé de préparation

La présente invention a également pour objet un procédé de préparation d'une composition cosmétique selon l'invention, comprenant :

- le mélange du gélifiant hydrophile avec l'eau, et optionnellement avec les agents émulsionnants hydrocarbonés et/ou siliconés, les humectants et les solvants solubles dans l'eau ;
- optionnellement l'ajout des agents filmogènes, des agents émulsionnants siliconés et/ou hydrocarbonés et des additifs ;
- l'ajout des nacres traités hydrophobes et optionnellement des pigments traités hydrophobes ;
- optionnellement l'ajustement du pH.

Procédé de maquillage des matières kératiniques

La présente invention concerne également un procédé de maquillage des matières kératiniques, en particulier la peau ou les lèvres, préférentiellement les paupières, consistant à appliquer sur lesdites matières kératiniques, en particulier sur la peau, ou les lèvres, préférentiellement les paupières, une composition cosmétique selon l'invention.

Dans un mode de réalisation préféré, le procédé de maquillage est un procédé de maquillage des paupières.

Utilisation cosmétique

La présente invention concerne également l'utilisation cosmétique non thérapeutique d'une composition selon l'invention, pour former, sur les matières kératiniques, en particulier sur la peau ou les lèvres, préférentiellement sur les paupières, un maquillage coloré à effet métallique aqueux.

Exemple 1 : Ombre à paupières

On a préparé une ombre à paupières (OAP1) ayant la composition présentée dans le tableau 1 suivant :

[Tableau 1]

| Nom INCI | Teneur (% en poids) |
|---|---|
| Nacres enrobées stéarate de magnésium | 24.60 |
| CARBOMER | 0.32 |
| POLYSORBATE 20 | 0.24 |
| GLYCERIN | 3.00 |
| BUTYLENE GLYCOL | 3.00 |
| ALCOHOL | 5.00 |
| CAPRYLYL GLYCOL | 0.25 |
| CHLORPHENESIN | 0.27 |
| ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL (Granresin SIW-MQIZ) | 12.00 dont 5.34% correspondant à l'extrait sec de trimethylsiloxysilicate |
| PEG-10 DIMETHICONE | 0.80 |
| AQUA | 50.13 |
| Solution de soude à 25% | 0.34 |

On a mélangé l'eau, le CARBOMER, le POLYSORBATE 20, la GLYCÉRINE, le BUTYLENE GLYCOL ainsi que le CAPRYLYL GLYCOL et le CHLORPHENESIN sous agitation dans un agitateur à hélices à 350 rpm.

L'ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL et le PEG-10 DIMETHICONE ont par la suite été incorporés, sous agitation dans un agitateur à hélices à 500 rpm.

On a laissé homogénéiser le mélange ainsi obtenu durant 15 minutes.

Les nacres enrobées stéarate de magnésium ont ensuite été introduites, toujours sous agitation à 500 rpm, jusqu'à obtenir une texture lisse et homogène.

L'ALCOHOL est enfin introduit et on laisse le mélange s'homogénéiser durant environ 10 minutes.

Le pH est ajusté à l'aide d'une solution de soude.

Exemple 2 : Ombres à paupières

On a préparé une ombre à paupières (OAP2) ayant la composition présentée dans le tableau 2 suivant :

[Tableau 2]

| Nom INCI | Teneur (% en poids) |
|---|---|
| Nacres enrobées stéarate de magnésium | 24.60 |
| CARBOMER | 0.32 |
| POLYSORBATE 20 | 0.24 |
| GLYCERIN | 3.00 |
| BUTYLENE GLYCOL | 3.00 |
| ALCOHOL | 5.00 |
| PHENOXYETHANOL | 0.75 |
| ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL (Granresin SIW-MQIZ) | 12.00 dont 5.34% correspondant à l'extrait sec de trimethylsiloxysilicate |
| PEG-10 DIMETHICONE | 0.80 |
| AQUA | 49.84 |

(suite)

| Nom INCI | Teneur (% en poids) |
|---|---|
| Solution de soude à 25% | 0.45 |

Un pré-mélange de CARBOMER et d'eau a été réalisé. Le POLYSORBATE 20, la GLYCERIN, le BUTYLENE GLYCOL, l'ALCOHOL et le PHENOXYETHANOL ont été mélangés au pré-mélange sous agitation.

L'ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL et le PEG-10 DIMETHICONE ont par la suite été introduits dans le mélange précédemment obtenu.

Les nacres enrobées stéarate de magnésium ont par la suite été ajoutées.

Le pH est ajusté à l'aide d'une solution de soude.

Exemple 3 : Ombres à paupières

Dans les exemples suivants, 4 ombres à paupières, respectivement OAP3, OPA4, OAP5, OAP6 ont été formulées en faisant varier différents paramètres tels que :

- la teneur en nacres enrobées stéarate de magnésium ;
- le traitement des nacres ou l'absence de traitement ;
- la teneur en pigments et leur traitement ou non.

Les différentes formules sont présentées dans le tableau ci-après :

[Tableau 3]

| Nom INCI | Teneur (% en poids) | | | |
|---|---|---|---|---|
| | OAP 3 | OAP 4 | OAP 5 | OAP 6 |
| Nacres enrobées stéarate de magnésium | 22.71 | - | - | 5.00 |
| Nacres non traitées | - | 24.60 | 20.30 | - |
| Oxyde de fer enrobé stéarate de magnésium | 1.87 | - | - | 19.58 |
| Oxyde de fer non traité | - | - | 4.35 | - |
| CARBOMER | 0.32 | 0.32 | 0.32 | 0.32 |
| POLYSORBATE 20 | 0.24 | 0.24 | 0.24 | 0.24 |
| GLYCERIN | 3.00 | 3.00 | 3.00 | 3.00 |
| BUTYLENE GLYCOL | 3.00 | 3.00 | 3.00 | 3.00 |
| ALCOHOL | 5.00 | 5.00 | 5.00 | 5.00 |
| PHENOXYETHANOL | 0.75 | 0.75 | 0.75 | 0.75 |
| ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL (Granresin SIW-MQIZ) | 12.00 dont 5.34% correspondant à l'extrait sec de trimethylsiloxysilicate | | | |
| PEG-10 DIMETHICONE | 0.80 | 0.80 | 0.80 | 0.80 |
| Solution de soude à 25% | 0.34 | 0.45 | 0.45 | 0.38 |
| AQUA | 49.97 | 49.79 | 49.79 | 49.92 |

Pour OAP3, OAP4, OAP5, un pré-mélange de CARBOMER et d'eau a été réalisé. Le POLYSORBATE 20, la GLYCERIN, le BUTYLENE GLYCOL, l'ALCOHOL et le PHENOXYETHANOL ont été mélangés au pré-mélange sous agitation.

L'ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL et le PEG-10 DIMETHICONE ont par la suite été introduits dans le mélange précédemment obtenu.

Ont ensuite été ajoutés :

- Les nacres enrobées stéarate de magnésium et l'oxyde de fer enrobé stéarate de magnésium pour OAP3;
- les nacres non traitées pour OAP4 ;
- les nacres non traitées et l'oxyde de fer non traité pour OAP5.

Le pH est ajusté à l'aide d'une solution de soude.

Pour l'OAP6, un pré-mélange de CARBOMER, de GLYCERINE, de BUTYLENE GLYCOL et d'eau est préparé.

Le POLYSORBATE 20, l'ALCOHOL et le PHENOXYETHANOL ont ensuite été mélangés au pré-mélange sous agitation.

L'ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL et le PEG-10 DIMETHICONE ont par la suite été introduits dans le mélange précédemment obtenu.

Les nacres enrobées stéarate de magnésium et l'oxyde de fer enrobé stéarate de magnésium ont enfin été introduits.

Le pH est ajusté à l'aide d'une solution de soude.

Exemple 4 : Evaluation de l'effet métallique

Mesure et quantification par Réflexion au Samba@

La brillance des formules a été évaluée au moyen d'un appareil Samba@ commercialisé par la société BOSSA NOVA TECHNOLOGIES (Venice, USA), méthode particulièrement connue de l'Homme du métier dans le domaine de la cosmétique pour l'évaluation de la brillance.

Le système Samba® est basé sur l'utilisation d'une caméra polarisée permettant l'évaluation de l'apparence visuelle en termes de brillance, de couleur et de diffusion de la lumière sur différents supports. Il s'agit d'une mesure sans contact qui permet d'effectuer des mesures immédiatement après application des produits (sans séchage).

Le système est composé de trois éléments principaux :

- Une caméra couleur de polarisation
- Une illumination polarisée
- Un support cylindrique.

La scène observée par la caméra de polarisation correspond à la carte d'application et au produit placé sur la partie cylindrique de montage, de - 12,7mm<x<12,7mm et -45°<thêta<45°. Ce système est équivalent à la mesure goniométrique grâce au support cylindrique.

La polarisation de la caméra couleur et de l'illuminant permet de décomposer la lumière diffusée en lumière spéculaire et diffuse pour chaque pixel de l'image. L'analyse de la couleur permet une décomposition de la lumière spéculaire dans les composantes de brillance et de chrominance.

La source envoie un faisceau lumineux polarisé sur l'échantillon. Lorsque ce faisceau entre en contact avec la surface, celui-ci est renvoyé vers la caméra en plusieurs composantes lumineuses :

- La première composante est la brillance. C'est une réflexion blanche polarisée de surface qui porte la même polarisation que la lumière incidente. Elle apparait comme une bande sur le produit. La largeur de la bande est déterminée par la rugosité de la surface et les irrégularités du produit et/ou de l'application.
- La deuxième composante est appelée la bande de chrominance (chroma).
- La troisième composante est la lumière diffusée.

Il est possible de visualiser et d'enregistrer les différentes images (somme, spéculaire, Shine, chroma et diffusé) et les profils spectraux angulaires correspondants.

Les données numériques sont calculées, permettant une caractérisation complète de l'aspect visuel du produit. Les mesures sont réalisées sur l'image ou sur la courbe.

Typiquement, plus le pic de réflectance (lumière spéculaire) est grand, plus le produit est brillant.

Calcul de l'intégrale spéculaire et du Wvisual

La première étape consiste en l'acquisition des images de l'échantillon.
La seconde étape consiste en l'extraction des distributions angulaires et le recueillement :

- du profil spéculaire ;
- du profil diffusé ;
- et la géométrie de la bande de brillance.

Ces profils sont, dans une troisième étape, caractérisés.
Le profil spéculaire permet d'obtenir les informations suivantes :

- le maximum du profil spéculaire ;
- l'intégrale spéculaire
- la largeur de la bande à mi-hauteur.

Le profil diffusé permet d'obtenir les observations suivantes :

- le maximum du profil de diffusion ;
- l'intégrale diffusée.

Le Wvisual en degrés se calcule de la manière suivante :
l'image est découpée en 10 puis sur chaque partie d'image on trace la courbe de réflexion et on calcule la largeur à mi-hauteur du pic. On réalise ensuite une moyenne sur les 10 courbes.

[Math1]

Wvisual (deg) = largeur à mi-hauteur du pic au Max/2 du pic de réflectance (lumière spéculaire) mesuré sur l'image,

Max : maximum du profil
Intégral : aire sous la courbe de réflectance (lumière spéculaire).

Typiquement, plus le Wvisual est petit, plus le produit est brillant.
Les réglages suivants ont été utilisés pour réaliser les évaluations de brillance des formules sur l'appareil Samba@ commercialisé par la société BOSSA NOVA TECHNOLOGIES (Venice, USA) :

- application à l'applicateur pneumatique ;
- dépôt de 150 $\mu$m ;
- mesure à T0 à température ambiante T environ 20-22°C ;
- application sur cartes de contrastes noires de la société BYK (ref. 2810).

Evaluation de l'effet métallique aqueux des différentes ombres à paupières

Les différentes ombres à paupières ont été évaluées par la méthode Samba® telle que précédemment décrite afin de caractériser l'effet métallique selon la présente invention.
La présence ou non de nacres enrobées magnésium stéarate ainsi que d'oxyde de fer enrobé stéarate ou non est résumée dans le tableau ci-après :

[Tableau 4]

|  | Nacres / Pigments | | | |
|---|---|---|---|---|
|  | Nacres traitées | Nacres non traitées | Oxyde de fer traité | Oxyde de fer non traité |
|  | Teneur (% en poids) | | | |
| OAP1 | 24.60 | - | - | - |

(suite)

| | Nacres / Pigments | | | |
|---|---|---|---|---|
| | Nacres traitées | Nacres non traitées | Oxyde de fer traité | Oxyde de fer non traité |
| | Teneur (% en poids) | | | |
| OAP2 | 24.60 | - | - | - |
| OAP3 | 22.71 | - | 1.87 | - |
| OAP4 | - | 24.60 | - | - |
| OAP5 | - | 20.30 | - | 4.35 |
| OAP6 | 5.00 | - | 19.60 | - |

Le Wvisual mesuré sur l'image, le Max, l'intégrale mesurée sur la courbe ainsi que l'effet observé à l'œil nu sont répertoriés dans le tableau ci-après pour chacune des ombres à paupières.

[Tableau 5]

| | Specular | | | Effet observé à l'œil nu |
|---|---|---|---|---|
| | Wvisual (deg) Mesuré sur l'image | Max | Intégrale mesurée sur la courbe | |
| OAP1 | 9 | 260,48 | 6998 | effet métallique aqueux |
| OAP2 | 11 | 333,78 | 7253 | effet métallique aqueux |
| OAP3 | 6 | 140,47 | 5165,67 | effet métallique satiné |
| OAP4 | 6 | 212,28 | 4757 | effet métallique satiné |
| OAP5 | 3 | 257,63 | 1582 | effet satiné |
| OAP6 | 3 | 135,61 | 2272 | effet satiné |

Avantageusement, les compositions cosmétiques selon la présente invention et comprenant des nacres traitées hydrophobes permettent d'obtenir l'effet métallique désiré appelé effet métallique aqueux, ou effet miroir.

Exemple 5 : Ombre à paupière

On a préparé une ombre à paupières (OAP7) ayant la composition présentée dans le tableau 6 suivant :

[Tableau 6]

| Nom INCI | Teneur (% en poids) |
|---|---|
| Nacres enrobées stéarate de magnésium | 24.66 |
| CARBOMER | 0.32 |
| POLYSORBATE 20 | 0.24 |
| GLYCERIN | 3.00 |
| BUTYLENE GLYCOL | 3.00 |
| ALCOHOL | 5.00 |
| PHENOXYETHANOL | 0.75 |
| ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL (Granresin SIW-MQIZ) | 12.00 dont 5.34% correspondant à l'extrait sec de trimethylsiloxysilicate |
| PEG-10 DIMETHICONE | 0.80 |
| AQUA | 49.90 |

(suite)

| Nom INCI | Teneur (% en poids) |
|---|---|
| Solution de soude à 25% | 0.34 |

Un pré-mélange de CARBOMER et d'eau a été réalisé. Le POLYSORBATE 20, la GLYCERIN, le BUTYLENE GLYCOL, l'ALCOHOL et le PHENOXYETHANOL ont été mélangés au pré-mélange sous agitation.

L'ISODECANE & TRIMETHYLSILOXYSILICATE & AQUA & PROPANEDIOL & DECYLGLUCOSIDE & PHENOXYETHANOL & CAPRYLYL GLYCOL & HEXYLENE GLYCOL et le PEG-10 DIMETHICONE ont par la suite été introduits dans le mélange précédemment obtenu.

Les nacres enrobées stéarate de magnésium ont ensuite été ajoutées.

Le pH est ajusté à l'aide d'une solution de soude.

L'ombre à paupière OAP7 une fois obtenue a été testée afin d'évaluer la tenue de l'effet métallique obtenu. Les évaluations ont été réalisées par une expérimentatrice:

- immédiatement après application de la composition sur les paupières ;

- avant le repas à T3h30 ;

- en fin de journée à T7h30.

Des notes, entre 0 et 9 sont attribuées par l'expérimentatrice pour :

- la présence du produit ;

- l'homogénéité de l'OAP ;

- la migration de l'OAP ou diffusion de l'OAP dans le pli de la paupière ;

- la diffusion de l'OAP autour de l'œil.

A partir de ces scores, sont calculés :

- la moyenne aux trois temps ;

- le delta - Δ - pour chaque temps (T3h30, T7h30).

Les deltas sont ensuite exprimés en pourcentage (%) :

- % de perte de produit (tenue teinte) ;

- % de perte d'homogénéité ;

- % de diffusion du produit autour de l'œil ;

- % de migration.

Les résultats sont consignés dans le tableau 7 suivant :

[Tableau 7]

| | Application | Résultat maquillage | Tenue T3h30 | Tenue T7h30 |
|---|---|---|---|---|
| Commentaires | Facile à travailler ; Assez facile à rendre homogène. | Intense ; Homogène ; Ne diffuse pas sous l'œil. | S'estompe très légèrement ; Un peu moins homogène ; Peu de migration ; Ne diffuse pas sous l'œil. | Ne s'estompe pas plus ; Ne perd pas plus en homogénéité ; Peu de migration ; Ne diffuse pas sous l'œil. |

De manière générale, l'OAP7 selon l'invention a un bon temps de travail, permettant un rendu de maquillage homogène et un résultat intense. L'OAP s'estompe très légèrement au bout de 3h30, en perdant très légèrement en homogénéité, mais ne migre quasiment pas et ne diffuse pas sous l'œil.

Ainsi et avantageusement, les compositions cosmétiques en phase continue aqueuse selon la présente invention, comprenant des nacres traitées hydrophobes permettent d'obtenir l'effet métallique désiré appelé effet métallique aqueux, ou effet miroir et présentent une texture longue tenue, fraiche et confortable.

**Revendications**

1. Composition cosmétique comprenant, dans une phase continue aqueuse,

   - au moins un agent de coloration choisi parmi les nacres traitées hydrophobes ;
   - au moins un gélifiant hydrophile ;
   - de l'eau.

2. Composition cosmétique selon la revendication 1, **caractérisée en ce que** les nacres traitées hydrophobes sont présentes en une teneur allant de 16 à 35% en poids, de préférence de 20 à 33% en poids, par rapport au poids total de la composition.

3. Composition cosmétique selon l'une quelconque des revendications précédentes **caractérisée en ce que** le traitement hydrophobe est un traitement par savon métallique.

4. Composition cosmétique selon l'une quelconque des revendications précédentes **caractérisée en ce que** le savon métallique est un savon d'acides gras ayant de 12 à 22 atomes de carbone, et en particulier de 12 à 18 atomes de carbone.

5. Composition cosmétique selon l'une quelconque des revendications précédentes **caractérisée en ce que** le métal du savon métallique est du zinc ou du magnésium.

6. Composition cosmétique selon l'une quelconque des revendications précédentes **caractérisée en ce que** le savon métallique est choisi parmi le laurate de zinc, le stéarate de magnésium, le myristate de magnésium, le stéarate de zinc, et leurs mélanges, et de préférence, le savon métallique est du stéarate de magnésium.

7. Composition cosmétique selon l'une quelconque des revendications précédentes **caractérisée en ce que** le gélifiant hydrophile est choisi parmi les polysaccharides tels que les exsudats de micro-organismes, préférentiellement la gomme xanthane, les extraits de végétaux, préférentiellement le tremella fuciformis polysaccharide, les polymères d'acide acrylique, préférentiellement le carbomère, ou un de leurs mélanges

8. Composition cosmétique selon l'une quelconque des revendications précédentes, **caractérisée en ce que** le gélifiant hydrophile est présent en une teneur allant de 0.05 à 3% en poids, de préférence de 0.1 à 2% en poids, par rapport au poids total de la composition.

9. Composition cosmétique selon l'une quelconque des revendications précédentes, **caractérisée en ce qu'**elle comprend de 30 à 70% en poids d'eau, de préférence de 40 à 60% en poids, par rapport au poids total de la composition.

10. Composition cosmétique selon l'une quelconque des revendications précédentes, **caractérisée en ce que** la phase aqueuse comprend au moins un autre agent de coloration choisi parmi les pigments traités hydrophobes, la teneur en pigments traités hydrophobes par rapport au poids total de la composition étant inférieure à 1,5%.

11. Composition cosmétique selon l'une quelconque des revendications précédentes, **caractérisée en ce qu'**elle comprend un humectant, choisi parmi les polyols et de préférence choisi parmi le glycérol et le butylène glycol.

12. Composition cosmétique selon l'une quelconque des revendications précédentes, **caractérisée en ce qu'**elle comprend un agent filmogène choisi parmi un polymère filmogène dispersible en phase aqueuse, préférentiellement, une dispersion aqueuse de résines triméthylsiloxysilicate, un polymère filmogène liposoluble tel que les résines triméthylsiloxysilicate dispersées dans de l'isododécane, les copolymères acrylate/polytriméthylsiloxyméthacrylate comprenant une structure carbosiloxane dendrimère greffée sur un squelette vinylique et leurs mélanges.

13. Composition cosmétique selon l'une quelconque des revendications précédentes, **caractérisée en ce que** la composition comprend au moins une huile et au moins un agent émulsionnant, ladite composition se présentant sous la forme d'une émulsion huile-dans-eau.

14. Composition cosmétique selon la revendication 13, **caractérisée en ce que** l'huile est une huile volatile choisie parmi les huiles volatiles hydrocarbonées, les alcanes linéaires volatiles, et les huiles siliconées volatiles.

15. Composition cosmétique selon la revendication 13 ou 14, **caractérisée en ce que** la composition comprend moins de 5% d'huiles non volatiles et de préférence la composition est exempte d'huiles non volatiles.

16. Composition cosmétique selon l'une quelconque des revendications 13 ou 14, **caractérisée en ce que** l'émulsionnant est choisi parmi les émulsionnants siliconés, les émulsionnants hydrocarbonés, ou un de leurs mélanges.

17. Procédé de préparation d'une composition cosmétique selon l'une quelconque des revendications précédentes, comprenant :

 - le mélange du gélifiant hydrophile avec l'eau, et optionnellement avec les agents émulsionnants hydrocarbonés et/ou siliconés, les agents humectants et les solvants solubles dans l'eau ;
 - optionnellement l'ajout des agents filmogènes, des agents émulsionnants siliconés et/ou hydrocarbonés et des additifs ;
 - l'ajout des nacres traitées hydrophobes et optionnellement des pigments traités hydrophobes ;
 - optionnellement l'ajustement du pH.

18. Procédé de maquillage des matières kératiniques, en particulier la peau, ou les lèvres, préférentiellement les paupières, consistant à appliquer sur lesdites matières kératiniques, en particulier la peau, ou les lèvres, préférentiellement les paupières, une composition cosmétique selon l'une quelconque des revendications 1 à 16.

19. Utilisation cosmétique d'une composition selon l'une quelconque des revendications 1 à 16, pour former, sur les matières kératiniques, en particulier sur la peau ou les lèvres, préférentiellement sur les paupières, un maquillage coloré à effet métallique.

**Patentansprüche**

1. Kosmetische Zusammensetzung, umfassend in einer wässrigen kontinuierlichen Phase

 - wenigstens ein Färbemittel, das gewählt ist aus hydrophob behandelten Perlmuttpartikeln;
 - wenigstens ein hydrophiles Geliermittel
 - Wasser.

2. Kosmetische Zusammensetzung nach Anspruch 1, **dadurch gekennzeichnet, dass** die hydrophob behandelten Perlmuttpartikel in einem Anteil von 16 bis 35 Gew.-%, bevorzugt 20 bis 33 Gew.-%, bezogen auf das Gesamtgewicht der Zusammensetzung, vorliegen.

3. Kosmetische Zusammensetzung nach einem der vorhergehenden Ansprüche, **dadurch gekennzeichnet, dass** die hydrophobe Behandlung eine Behandlung mit Metallseife ist.

4. Kosmetische Zusammensetzung nach einem der vorhergehenden Ansprüche, **dadurch gekennzeichnet, dass** die Metallseife eine Seife aus Fettsäuren mit 12 bis 22 Kohlenstoffatomen und insbesondere 12 bis 18 Kohlenstoffatomen ist.

5. Kosmetische Zusammensetzung nach einem der vorhergehenden Ansprüche, **dadurch gekennzeichnet, dass** das Metall der Metallseife Zink oder Magnesium ist.

6. Kosmetische Zusammensetzung nach einem der vorhergehenden Ansprüche, **dadurch gekennzeichnet, dass** die Metallseife gewählt ist aus Zinklaurat, Magnesiumstearat, Magnesiummyristat, Zinkstearat und Mischungen davon, und bevorzugt die Metallseife Magnesiumstearat ist.

7. Kosmetische Zusammensetzung nach einem der vorhergehenden Ansprüche, **dadurch gekennzeichnet, dass** das hydrophile Geliermittel gewählt ist aus Polysacchariden wie Exsudaten von Mikroorganismen, bevorzugt Xanthangummi, Pflanzenextrakten, bevorzugt Tremella fuciformis-Polysaccharid, Acrylsäurepolymeren, bevorzugt Carbomer, oder einem Gemisch davon.

8. Kosmetische Zusammensetzung nach einem der vorhergehenden Ansprüche, **dadurch gekennzeichnet, dass** der hydrophile Gelbildner in einem Gehalt von 0,05 bis 3 Gew.-%, bevorzugt 0,1 bis 2 Gew.-%, bezogen auf das Gesamtgewicht der Zusammensetzung, vorhanden ist.

9. Kosmetische Zusammensetzung nach einem der vorhergehenden Ansprüche, **dadurch gekennzeichnet, dass** sie 30 bis 70 Gew.-% Wasser, bevorzugt 40 bis 60 Gew.-%, bezogen auf das Gesamtgewicht der Zusammensetzung, umfasst.

10. Kosmetische Zusammensetzung nach einem der vorhergehenden Ansprüche, **dadurch gekennzeichnet, dass** die wässrige Phase wenigstens ein weiteres Färbemittel enthält, das unter den hydrophob behandelten Pigmenten gewählt ist, wobei der Gehalt an hydrophob behandelten Pigmenten, bezogen auf das Gesamtgewicht der Zusammensetzung, weniger als 1,5 % beträgt.

11. Kosmetische Zusammensetzung nach einem der vorhergehenden Ansprüche, **dadurch gekennzeichnet, dass** sie ein Feuchthaltemittel enthält, das unter den Polyolen gewählt ist und bevorzugt aus Glycerin und Butylenglycol gewählt ist.

12. Kosmetische Zusammensetzung nach einem der vorhergehenden Ansprüche, **dadurch gekennzeichnet, dass** sie ein filmbildendes Mittel enthält, das gewählt ist aus einem in wässriger Phase dispergierbaren filmbildenden Polymer, bevorzugt einer wässrigen Dispersion von Trimethylsiloxysilikatharzen, einem fettlöslichen filmbildenden Polymer, wie in Isododecan dispergierte Trimethylsiloxysilikatharze, Acrylat/Polytrimethylsiloxymethacrylat-Copolymere, die eine dendrimere Carbosiloxanstruktur umfassen, die auf ein Vinylgerüst aufgepfropft ist, und deren Mischungen.

13. Kosmetische Zusammensetzung nach einem der vorhergehenden Ansprüche, **dadurch gekennzeichnet, dass** die Zusammensetzung wenigstens ein Öl und wenigstens einen Emulgator enthält, wobei die Zusammensetzung in Form einer Ölin-Wasser-Emulsion vorliegt.

14. Kosmetische Zusammensetzung nach Anspruch 13, **dadurch gekennzeichnet, dass** das Öl ein flüchtiges Öl ist, das unter den flüchtigen Kohlenwasserstoffölen, den flüchtigen linearen Alkanen und den flüchtigen Silikonölen gewählt ist.

15. Kosmetische Zusammensetzung nach Anspruch 13 oder 14, **dadurch gekennzeichnet, dass** die Zusammensetzung weniger als 5 % nichtflüchtige Öle enthält und die Zusammensetzung bevorzugt frei von nichtflüchtigen Ölen ist.

16. Kosmetische Zusammensetzung nach einem der Ansprüche 13 oder 14, **dadurch gekennzeichnet, dass** der Emulgator aus Silikonemulgatoren, Kohlenwasserstoffemulgatoren oder deren Mischungen gewählt ist.

17. Verfahren zur Herstellung einer kosmetischen Zusammensetzung nach einem der vorhergehenden Ansprüche, umfassend:

- Mischen des hydrophilen Gelbildners mit Wasser und optional mit den Kohlenwasserstoff- und/oder Silikonemulgatoren, Feuchthaltemitteln und wasserlöslichen Lösungsmitteln;
- optional die Zugabe der Filmbildner, der Silikon- und/oder KohlenwasserstoffEmulgatoren und der Zusatzstoffe,
- Zugabe von hydrophob behandelten Perlmuttpartikeln und optional von hydrophob behandelten Pigmenten;
- optional das Einstellen des pH-Werts.

18. Verfahren zum Schminken von Keratinmaterialien, insbesondere der Haut oder der Lippen, bevorzugt der Augenlider, das darin besteht, auf die Keratinmaterialien, insbesondere die Haut oder die Lippen, bevorzugt die Augenlider, eine kosmetische Zusammensetzung nach einem der Ansprüche 1 bis 16 aufzutragen.

19. Kosmetische Verwendung einer Zusammensetzung nach einem der Ansprüche 1 bis 16, um auf Keratinmaterialien, insbesondere auf der Haut oder den Lippen, bevorzugt auf den Augenlidern, ein farbiges Make-up mit Metalleffekt zu bilden.

**Claims**

1. A cosmetic composition comprising, in a continuous aqueous phase,

   - at least one colouring agent chosen from hydrophobic treated nacres;
   - at least one hydrophilic gelling agent;
   - water.

2. The cosmetic composition according to claim 1, wherein the hydrophobic treated nacres are present with a content ranging from 16 to 35% by weight, preferably from 20 to 33% by weight, with respect to the total weight of the composition.

3. The cosmetic composition according to any one of the preceding claims, wherein the hydrophobic treatment is a treatment by metallic soap.

4. The cosmetic composition according to any one of the preceding claims, wherein the metallic soap is a fatty acid soap having 12 to 22 carbon atoms, and in particular 12 to 18 carbon atoms.

5. The cosmetic composition according to any one of the preceding claims, wherein the metal of the metallic soap is zinc or magnesium.

6. The cosmetic composition according to any one of the preceding claims, wherein the metallic soap is chosen from zinc laurate, magnesium stearate, magnesium myristate, zinc stearate and the mixtures thereof, and the metallic soap is preferably magnesium stearate.

7. The cosmetic composition according to any one of the preceding claims, wherein the hydrophilic gelling agent is chosen from the polysaccharides such as the exudates of microorganisms, preferably xanthan gum, plant extracts, preferably tremella fuciformis polysaccharide, acrylic acid polymers, preferably carbomer, or one of the mixtures thereof.

8. The cosmetic composition according to any one of the preceding claims, wherein the hydrophilic gelling agent is present with a content ranging from 0.05 to 3% by weight, preferably from 0.1 to 2% by weight, with respect to the total weight of the composition.

9. The cosmetic composition according to any one of the preceding claims, comprising 30 to 70% by weight water, preferably 40 to 60% by weight, with respect to the total weight of the composition.

10. The cosmetic composition according to any one of the preceding claims, wherein the aqueous phase comprises at least one other colouring agent chosen from the hydrophobic treated pigments, the content of hydrophobic treated pigments with respect to the total weight of the composition being less than 1.5%.

11. The cosmetic composition according to any one of the preceding claims, further comprising a humectant, chosen from the polyols and preferably chosen from glycerol and butylene glycol.

**12.** The cosmetic composition according to any one of the preceding claims, further comprising a film-forming agent chosen from a film-forming polymer dispersible in the aqueous phase, preferably an aqueous dispersion of trimethylsiloxysilicate resins, a liposoluble film-forming polymer such as trimethylsiloxysilicate resins dispersed in isododecane, acrylate/polytrimethylsiloxymethacrylate copolymers comprising a carbosiloxane dendrimer structure grafted on a vinyl backbone and the mixtures thereof.

**13.** The cosmetic composition according to any one of the preceding claims, further comprising at least one oil and at least one emulsifier, said composition being in the form of an oil-in-water emulsion.

**14.** The cosmetic composition according to claim 13, wherein the oil is a volatile oil chosen from volatile hydrocarbon oils, volatile linear alkanes and volatile silicon oils.

**15.** The cosmetic composition according to claim 13 or 14, wherein the composition comprises less than 5% non-volatile oils and, preferably, the composition is free of non-volatile oils.

**16.** The cosmetic composition according to claim 13 or 14, wherein the emulsifier is chosen from silicone emulsifiers, hydrocarbon emulsifiers or the mixtures thereof.

**17.** A method for preparing a cosmetic composition according to any one of the preceding claims, comprising:

mixing the hydrophilic gelling agent with water, and optionally with the hydrocarbon and/or silicone emulsifiers, the humectants and the water-soluble solvents;
optionally adding film-forming agents, silicone and/or hydrocarbon emulsifiers and additives;
adding hydrophobic treated nacres and optionally hydrophobic treated pigments; and
optionally adjusting the pH.

**18.** A method for making up keratin materials, in particular the skin or the lips, preferably the eyelids, comprising applying on said keratin materials, in particular the skin or the lips, preferably the eyelids, a cosmetic composition according to any one of claims 1 to 16.

**19.** Use of a cosmetic composition according to any one of claims 1 to 16, to form on keratin materials, in particular on the skin or the lips, preferably on the eyelids, a coloured makeup with metallic effect.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2679771
- EP 1184426 A
- FR 2232303 A
- US 5162410 A
- WO 2004073626 A
- US 5874069 A
- US 5919441 A
- US 6051216 A
- US 5981680 A
- WO 20071068371 A
- WO 2008155059 A
- WO 200815505 A
- US 2004175338 A
- EP 847752 A